# EUROPÄISCHE PATENTANMELDUNG

(11) **EP 0 791 366 A1**
(43) Veröffentlichungstag der Anmeldung: **27.08.1997**
(21) Anmeldenummer: 96102707.5
(22) Anmeldetag: 23.02.1996
(51) Int. Cl.: A61M 1/00, F04B 27/04, F04B 27/053

(54) **Saugaggregat**

(71) Anmelder: Medela AG, 6340 Baar (CH)
(72) Erfinder: Larsson, Olaf, 6300 Zug (CH)
(74) Vertreter: Troesch Scheidegger Werner AG

(57) **Zusammenfassung**

Das Saugaggregat weist eine Mehrzahl sternförmig um eine zentrale Achse (A) angeordneter Zylinder-Kolben-Einheiten (1, 16; 2, 17; 3, 18; 4, 19) auf, welche in einem gemeinsamen Gehäuse (5) zusammengehalten sind. Die Zylinder (1-4) weisen Einlass- bzw. Auslassventile (20, 21; 22, 23 usw.) auf. Die Kolben (16-19) sind über Pleuel (11-14) an eine Kurbel angelenkt, wobei alle Pleuel (11-14) an eine gemeinsame Büchse (15) angelenkt sind, welche auf der Kurbel sitzt. Die Gelenke zwischen Pleuel und Kolben bzw. Büchse sind vorzugsweise als sog. Filmscharniere (S) ausgebildet, was möglich ist, wenn Kolben, Pleuel und Büchse aus demselben Kunststoff geformt sind.

## Beschreibung

Die vorliegende Erfindung betrifft ein Saugaggregat, d.h. ein als Pumpe arbeitendes Volumen-Förderaggregat, insbesondere zur Verwendung als Saugaggregat in Spitälern.

Bekannte Saugaggregate für medizinische Verwendung basieren auf verschiedenen mechanischen Grundprinzipien, sind jedoch in der Regel mit mehr oder weniger gravierenden Nachteilen behaftet, sei dies deren komplexer Aufbau, die Schwierigkeit bei der Reinigung oder der hohe Anschaffungspreis.

Aufgabe der vorliegenden Erfindung war die Schaffung eines neuartigen, einfach aufgebauten Saugaggregates.

Diese Aufgabe wird beim erfindungsgemässen Saugaggregat durch die Merkmale gemäss dem kennzeichnenden Teil von Anspruch 1 gelöst.

Bei einer besonders bevorzugten Ausführungsform bestehen sämtliche Bauteile des Aggregates aus Kunststoff, was deren Zusammenbau durch einfachstes Zusammenstecken ohne Zuhilfenahme von zusätzlichen Verbindungselementen (z.B. Schrauben) ermöglicht.

Dadurch entsteht ein besonders preisgünstiges Aggregat mit langer Lebensdauer. Der Einsatz für medizinische Anwendungen ist problemlos. Es ist grundsätzlich möglich, das Saugaggregat nach Verwendung bei einem Patienten wegzuwerfen, was eine problematische Reinigung bzw. Sterilisierung hinfällig werden lässt.

Weitere Details der Ausführung sind in den abhängigen Ansprüchen definiert.

Die Erfindung wird nachstehend anhand eines in der Zeichnung dargestellten Ausführungsbeispiels noch etwas näher erläutert. Es zeigen:

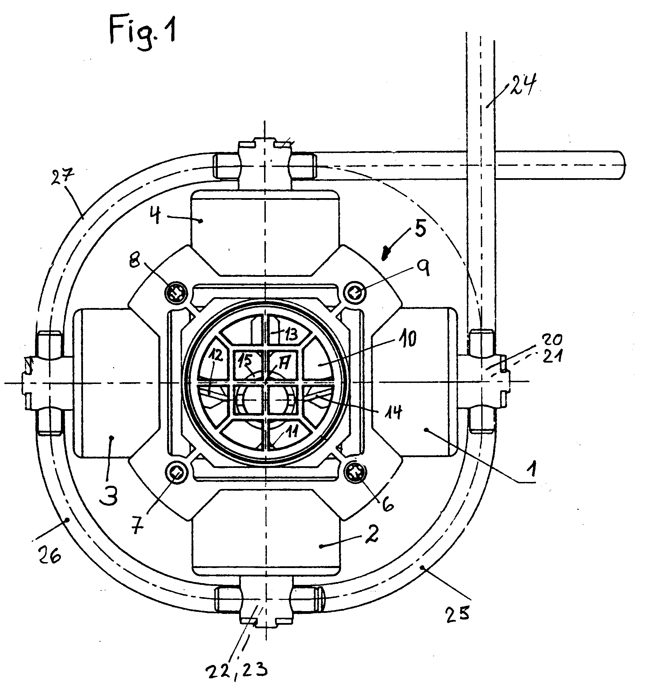

- Fig. 1: eine Stirnansicht eines Saugaggregates nach der Erfindung;
- Fig. 2: einen Vertikalschnitt durch das Aggregat nach Fig. 1;
- Fig. 3: einen Querschnitt entlang der Linie I-I von Fig. 1 und
- Fig. 4: einen Schnitt entlang der Linie II-II von Fig. 2.

Das in der Zeichnung dargestellte Saugaggregat weist vier sternförmig um die zentrale Achse A angeordnete Zylinder 1-4 auf, welche als Einheiten aus Kunststoff zusammengesteckt ein Gehäuse 5 bilden. Das Gehäuse 5 ist dazu vorgesehen, an einen nicht dargestellten Antriebsmotor angeflanscht zu werden. Mittels durch die Bohrungen 6-9 führenden Befestigungsschrauben (nicht dargestellt) können die beiden Gehäusehälften 5a, 5b bei Bedarf zusammengeschraubt werden.

Die Antriebswelle des Motors ist mit einem eine Kurbel bildenden Exzenter versehen, welcher in die zentrale Bohrung 10 ragt und auf welchem die Pleuel 11-14 der Kolben der Zylinder 1-4 gelagert sind. Diese Pleuel 11-14 sind an einer gemeinsamen Büchse 15 angelenkt, welche ihrerseits auf der Kurbel bzw. dem Exzenter angeordnet ist.

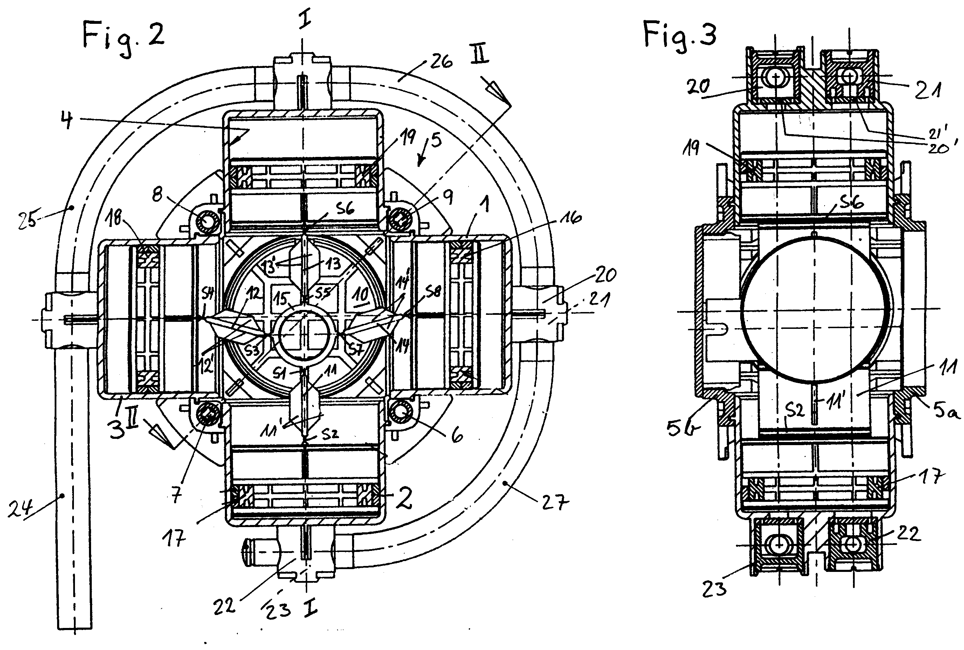

Jeder Zylinder weist neben einem Kolben 16-19 (siehe Fig. 2) je ein Einlass- und ein Auslassventil 20, 21 bzw. 22, 23 usw. auf (siehe Fig. 3).

Die an eine Saugleitung 24 anschliessbaren Einlassventile 20, 22 usw. sind untereinander über Leitungen 25-27 verbunden. Dasselbe gilt für die Auslassventile 21, 23 usw.

Das Kernstück des Saugaggregates liegt in den als steife Lamellen ausgebildeten Pleueln 11-14 (Versteifungen 11'-14'), welche aus demselben Kunststoff bestehen wie die Kolben 16-19 und die Büchse 15, welche über sog. Filmscharniere S1, S2; S3, S4; S5, S6 und S7, S8 an den Kolben bzw. der Büchse angelenkt sind.

Dank dieser Konstruktion kann auf besondere Gelenke zwischen Pleueln und Kolben einerseits sowie Pleueln und Büchse andererseits verzichtet werden, was wesentlich dazu beiträgt, die gesamte Konstruktion aus Kunststoff herzustellen, was wiederum die Montage durch Steck- bzw. Klemmverbindungen zwischen einzelnen Teilen ermöglicht.

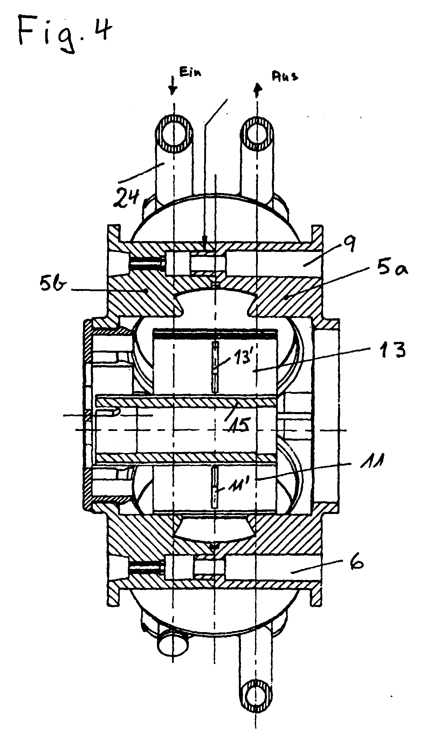

Details des Saugaggregates sind aus den Fig. 2-4 ersichtlich, wobei Fig. 2 den besten Gesamtüberblick erlaubt und insbesondere den neuartigen Antrieb zeigt.

Durch den Einsatz mehrerer Zylinder-Kolben-Einheiten kann eine gleichmässige Saugleistung erzielt werden.

Das die Zylinder zusammenhaltende Gehäuse besteht vorzugsweise aus zwei gegeneinander klemmbaren Hälften 5a, 5b (Fig. 3), welche die Zylinder zwischen sich festzuhalten vermögen.

Die Kolben 16-19 sind mit üblichen Dichtringen ausgerüstet und tragen dazu bei, eine optimale Saugleistung zu erzielen.

Als Ventile eignen sich Konstruktionen mit elastisch verformbaren Klappen an sich bekannter Bauart.

## Patentansprüche

1. Saugaggregat, gekennzeichnet durch eine Mehrzahl von sternförmig um eine zentrale Achse angeordnete, in einem gemeinsamen Gehäuse zusammengehaltene Zylinder-Kolben-Einheiten, deren Zylinder-Arbeitsräume über je mindestens ein Ein- und ein Auslassventil an Saug- bzw. Auslassleitungen anschliessbar sind und deren Pleuel von einer auf der genannten Achse liegenden, motorisch angetriebenen Kurbel bewegt werden.

2. Saugaggregat nach Anspruch 1, dadurch gekennzeichnet, dass die Kurbel von einem auf der Abtriebswelle eines Motors sitzenden Exzenter gebildet ist.

3. Saugaggregat nach einem der Ansprüche 1 oder 2, dadurch gekennzeichnet, dass im wesentlichen sämtliche Teile des Aggregates aus Kunststoff bestehen und vorzugsweise durch Zusammenstecken ohne Zuhilfenahme von zusätzlichen Verbindungselementen zusammengehalten sind.

4. Saugaggregat nach einem der Ansprüche 1 bis 3, dadurch gekennzeichnet, dass die Pleuel aus steifen Lamellen gebildet sind, welche am zugehörigen Kolben und an einer gemeinsamen, auf die Antriebskurbel aufzusetzenden Hülse angelenkt sind.

5. Saugaggregat nach Anspruch 4, dadurch gekennzeichnet, dass die Pleuel und die genannte Hülse aus demselben Kunststoffmaterial bestehen wie die Kolben und die Anlenkung der Pleuel an die Kolben und die Hülse über sog. Filmscharniere erfolgt.

6. Saugaggregat nach einem der Ansprüche 1-5, dadurch gekennzeichnet, dass die Einlassventile aller Zylinder über Verbindungsleitungen untereinander verbunden sind und an eine gemeinsame Saugleitung anschliessbar sind.

7. Saugaggregat nach einem der Ansprüche 1-5, dadurch gekennzeichnet, dass die Auslassventile aller Zylinder über Verbindungsleitungen untereinander verbunden sind und an eine gemeinsame Auslassleitung anschliessbar sind.
